# EUROPÄISCHE PATENTSCHRIFT

(11) **EP 1 410 754 B1**
(45) Veröffentlichungstag und Bekanntmachung des Hinweises auf die Patenterteilung: **24.05.2006**
(21) Anmeldenummer: 02023052.0
(22) Anmeldetag: 16.10.2002
(51) Int. Cl.: A61B 3/135, G02B 21/00

(54) **Operationsmikroskop mit einer Beleuchtungseinrichtung**
Surgical microscope with illumination means
Microscope chirurgical à dispositif d'illumination

(43) Veröffentlichungstag der Anmeldung: 21.04.2004
(73) Patentinhaber: Möller-Wedel GmbH, 22880 Wedel (DE)
(72) Erfinder: Koetke, Jochen, Dr., 22083 Hamburg (DE)
(74) Vertreter: Glawe, Delfs, Moll

(56) Entgegenhaltungen:
- DE-A- 19 856 847
- US-A- 5 135 299
- US-A- 5 943 118
- PATENT ABSTRACTS OF JAPAN vol. 004, no. 168 (P-037), 20. November 1980 (1980-11-20) & JP 55 115013 A (INAMI:KK), 4. September 1980 (1980-09-04)

## Beschreibung

Die Erfindung betrifft ein Operationsmikroskop mit einer die Objektebene mit einem Lichtfleck beleuchtenden, hinter der Frontlinse angeordneten Beleuchtungseinrichtung, in deren Strahlengang eine denselben teilweise abdeckende, bewegbare Blende angeordnet ist. Ein solches Operations mikroskop ist bekannt aus DE-A-198 56 847.

Eine solche Anordnung bildet die Blende, z. B. eine Spaltblende, präzise durch die Frontlinse hindurch in die Objektebene der Frontlinse ab und erzeugt dort einen Lichtfleck, im Falle einer Spaltblende einen im wesentlichen rechteckigen, länglichen Lichtfleck.

Die sogenannte Spaltbeleuchtung oder Spaltlampe ist unverzichtbares Hilfsmittel des Augenarztes in der Diagnose vieler Augenkrankheiten, weil sich mit dieser Beleuchtung in Form eines feinen, scharf begrenzten Lichtflecks die inneren nahezu transparenten Strukturen des zu untersuchenden Auges sozusagen im Schnitt darstellen und bewerten lassen. Daher wird die Spaltbeleuchtung in Operationsmikroskopen für die Augenchirurgie eingesetzt, hier auch zur Schaffung kontrastreicher Beleuchtung für die schwierige optische Kontrolle der Rhexisführung bei der Operation besonders dichter Katarakte.

Bei operativen Eingriffen im hinteren Augenabschnitt, beispielsweise an der Netzhaut, wird die Spaltbeleuchtung zur Darstellung feinster Membranen eingesetzt, wobei das Operationsmikroskop üblicherweise um einen optischen Vorsatz zur Kompensation der optischen Wirkung des operierten Auges erweitert wird, das vor bzw. unter der Frontlinse angebracht wird und im Mikroskopokular ein optisch scharfes Bild von Strukturen innerhalb des Auges erzeugt.

Es ist zwar auch bekannt, die Beleuchtungseinrichtung unterhalb bzw. vor der Frontlinse vorzusehen. Die Beleuchtung durch die Frontlinse mit hinter der Frontlinse angeordneter Beleuchtungseinrichtung ermöglicht jedoch eine kompakte Bauform des Mikroskops und einen nur geringen Winkel zwischen den optischen Achsen der Beobachtungsstrahlengänge des Mikroskops und dem Beleuchtungsstrahlengang, um in der Netzhautchirurgie Bereiche der Netzhaut beleuchten zu können, die durch das Mikroskop hindurch beobachtbar sind.

Bei einem bekannten Operationsmikroskop der eingangs genannten Art mit Spaltbeleuchtung durch die Frontlinse hindurch ist die in die Objektebene abgebildete Spaltblende höchstens um die Achse des Beleuchtungsstrahlenganges drehbar.

Die Aufgabe der Erfindung besteht in der Schaffung eines Operationsmikroskops mit einer verbesserten Beleuchtung des Operationsfeldes, die eine größere Variabilität aufweist und mit der eine für Operationen bessere Beleuchtung erreicht werden kann.

Die Lösung der Aufgabe besteht darin, daß der Lichtfleck mit einer translatorischen Bewegungskomponente in der Objektebene verschiebbar ist.

Die Blende wird also nicht wie beim Stand der Technik nur um die optische Achse des Beleuchtungsstrahlenganges gedreht, was eine entsprechende Drehung des Lichtflecks mit sich bringt. Vielmehr wird der Lichtfleck relativ zur Mitte des Bildfelds verschoben, so daß im Falle einer Spaltblende das Operationsfeld mit einem "Lichtbalken", d. h. einem Lichtfleck abgetastet werden kann, der die Form eines länglichen Rechtecks hat. "Abtasten" bedeutet in diesem Falle auch, daß diese Abtastbewegung nicht kontinuierlich erfolgen muß, sondern an einer Stelle angehalten werden kann, die für die Operation besonders vorteilhaft ist.

Bei einer vorteilhaften Ausführungsform ist die Blende für eine Bewegung mit einer im Strahlengang translatorischen Komponente senkrecht zur optischen Achse des Beleuchtungsstrahlenganges ausgebildet. Auch der Lichtbalken bzw. Lichtfleck wird dann im wesentlichen eine translatorische Bewegung durch das Bildfeld ausführen.

Alternativ kann aber auch die Beleuchtungseinrichtung relativ zur Blende bewegbar sein, was eine ähnliche Wirkung erzeugt. Bei einer anderen Ausführungsform kann statt dessen oder zusätzlich der Lichtfleck durch Verschwenken eines Umlenkelements für das Beleuchtungslicht verschiebbar sein.

Bei einer vorteilhaften Ausführungsform ist die Blende, genauer gesagt die Blendenöffnung, in einem senkrecht zur optischen Achse des Beleuchtungsstrahlenganges verschiebbaren Blendenträger angeordnet. Sie kann also exakt senkrecht zur optischen Achse verschoben werden. Es ist auch möglich, diese Verschiebbarkeit in zwei zueinander senkrechten Richtungen durch entsprechende Gleitführungen zu bewirken. Zusätzlich kann noch vorgesehen sein, daß die Blende um eine Achse gedreht werden kann, die parallel zur optischen Achse des Beleuchtungsstrahlenganges ist.

Statt eines verschiebbaren Blendenträgers kann auch ein Blendenträger vorgesehen sein, der exzentrisch zur optischen Achse des Beleuchtungsstrahlenganges drehbar gelagert ist. Auch in diesem Falle führt die Blendenöffnung im wesentlichen eine translatorische Bewegung durch, die allerdings von einer Drehbewegung überlagert ist. Wie groß die Drehbewegung im Vergleich zur translatorischen Bewegung ist, hängt dabei davon ab, wie stark exzentrisch der Blendenträger gelagert ist.

Auf dem Blendenträger kann mehr als eine Blendenöffnung vorgesehen sein. Z. B. könnten spaltförmige Blendenöffnungen mit unterschiedlichen Spaltbreiten angeordnet sein. Eine oder mehrere Blendenöffnungen könnten auch kreisförmig sein, diese würden die Objektebene bzw. das Auge zur Streulichtunterdrükkung exzentrisch zur Bildmitte beleuchten.

Statt mehrerer Blendenöffnungen unterschiedlicher Größen kann auch vorgesehen sein, daß die Größe der Blendenöffnung (die Spaltbreite bzw. der Kreisdurchmesser) veränderbar ist.

Bei einer anderen vorteilhaften Ausführungsform ist vorgesehen, daß die Blendenöffnung bzw. die Blendenöffnungen auf wenigstens in Teilbereichen teildurchlässigen Blendenträgern angeordnet ist/sind. In diesem Falle erhält man eine helle Beleuchtung im Bereich des Spaltbildes bzw. Kreisbildes und eine stark abgeschwächte Beleuchtung um dieses herum, was ebenfalls für viele Zwecke vorteilhaft sein kann.

Die Blende(n) und/oder das Umlenkelement kann/können durch entsprechende Hebel von Hand verstellbar sein. Bei einer vorteilhaften Ausführungsform geschieht diese Verstellung aber motorisch und kann beispielsweise über einen Fußschalter vom Anwender bedient werden.

Die Erfindung wird im folgenden anhand von vorteilhaften Ausführungsformen unter Bezugnahme auf die beigefügten Zeichnungen beschrieben. Es zeigen:
- Fig. 1: in schematischer Ansicht in einem seitlichen Schnitt Teile des Mikroskops und die Beleuchtungseinrichtung in einer ersten Ausführungsform;
- Fig. 2: in ähnlicher Ansicht wie in Fig. 1 eine andere Ausführungsform des Mikroskops mit der erfindungsgemäßen Beleuchtungseinrichtung;
- Fig. 3: einen ersten Blendenträger der Erfindung mit seinen Bewegungsmöglichkeiten;
- Fig. 4: einen zweiten Blendenträger der Erfindung mit seinen Bewegungsmöglichkeiten;
- Fig. 5: eine schematische Darstellung des Auges mit dem beleuchtenden Lichtbalken; und
- Fig. 6: in schematischer Ansicht eine Anordnung, mit der die Bewegungsmöglichkeiten der Fig. 3 erreicht werden können.

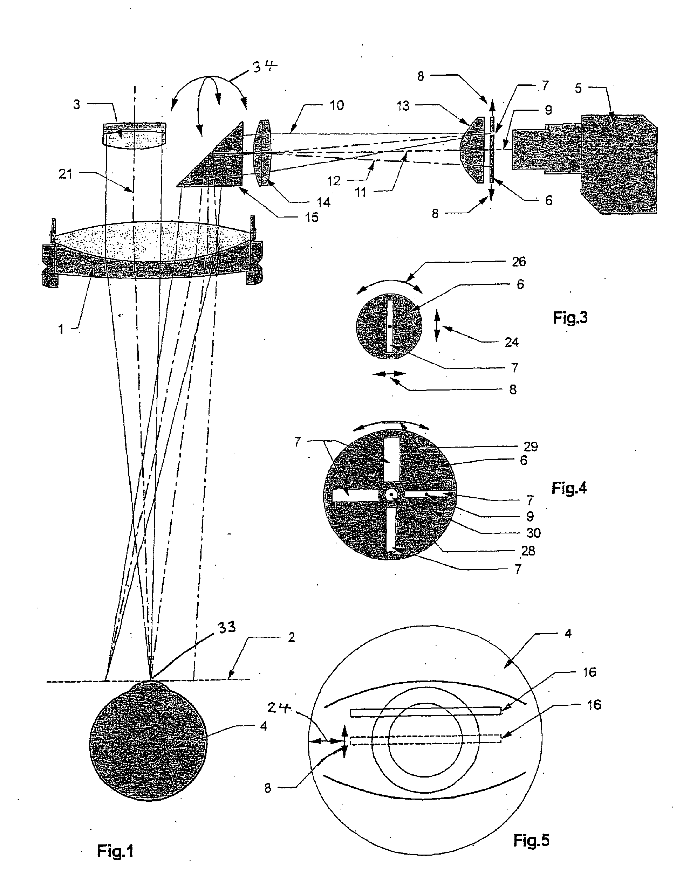

Das in den Fig. 1 und 2 gezeigte Mikroskop weist eine Frontlinse 1 auf, durch das die Beobachtung der Bildebene 2 mit weiteren optischen Elementen möglich ist, von denen eines bei 3 angedeutet ist. Mit einem solchen Mikroskop kann z. B. ein menschliches Auge 4 beobachtet werden. Ebenfalls in den Figuren 1 und 2 ist eine Beleuchtungseinrichtung gezeigt. Als Lichtquelle dient hier z. B. das Ende 5 eines Lichtleiters. Das Licht trifft auf einen Blendenträger 6 mit einer spaltförmigen Blendenöffnung 7. Der Blendenträger 6 mit der Blendenöffnung 7 ist in Richtung der Pfeile 8, wie dies auch in Fig. 3 angedeutet ist, senkrecht zur Achse 9 des Beleuchtungsstrahlenganges und senkrecht zu seiner Längserstreckung verschiebbar. Man erhält in der in den Fig. 1 und 2 gezeigten Stellung des Spaltes 7 den mit ausgezogenen Linien gezeigten Strahlengang 10, der noch durch optische Elemente 13, 14, 15 abgebildet und umgelenkt wird sowie durch die Frontlinse 1 auf die Objektebene 2 abgebildet wird. Bei anderen Stellungen des Spaltes 7 erhält man Strahlengänge, von denen jeweils nur der Mittelstrahl 11 bzw. 12 dargestellt ist. Dadurch erhält man, wie dies in Fig. 5 gezeigt ist, auf dem Auge 4 einen strich- oder balkenförmigen Lichtfleck 16, von dem zwei Stellungen gezeigt sind und der in Richtung des Doppelpfeiles 8 relativ zur Mitte 33 des Bildfeldes verschiebbar ist. Der Lichtbalken 16 ist also auf der Cornea des Patientenauges 4 seitlich verschiebbar, um etwa bei Kataraktoperationen die Position mit optimalem Beleuchtungskontrast einzustellen. Anstelle der Verschiebung des Spaltes 7 oder zusätzlich dazu kann das Umlenkelement 15 in Richtung der Doppelpfeile 34 verschwenkt werden.

In Fig. 2 ist im wesentlichen der gleiche Aufbau der Beleuchtungseinrichtung wie bei der Ausführungsform der Fig. 1 gezeigt. Diese Ausführungsform ist vorteilhaft zur Beleuchtung des hinteren Abschnitts des Auges 4. Es ist ein optischer Vorsatz 17 vor bzw. unter der Frontlinse zur Kompensation der optischen Wirkung des operierten Auges vorgesehen. Solche Vorsatzsysteme 17 bestehen häufig aus einer Linse 18 kurzer Brennweite, die die Netzhaut des Auges 4 oder Bereiche vor der Netzhaut in eine Zwischenbildebene 19 abbildet. Dieses Zwischenbild wird durch das Mikroskop beobachtet, gegebenenfalls mit zusätzlichen Optiken 20 zur Anpassung des optischen Beobachtungsstrahlenganges 21 des Mikroskops an das Zwischenbild und/oder zur höhen- und seitenrichtigen Abbildung. Durch die Linse 18 wird das Beleuchtungslicht scharf auf die entsprechende Stelle der Netzhaut abgebildet. Durch Verändern der Stellung der Blendenöffnung oder Verschwenken des Umlenkelementes 15 überstreicht der Lichtbalken oder anders geformte Lichtfleck praktisch den gesamten durch das Mikroskop einsehbaren Bereich der Netzhaut und des Glaskörpers und ermöglicht eine Bewertung des Gewebes.

Wie dies aus Fig. 2 ersichtlich ist, kann durch seitliche Verschiebung der Spaltblende 7 der Anwender durch die optische Wirkung der augennahen Linse 18 den gesamten, durch das Operationsmikroskop einsehbaren Netzhautbereich mit dem Lichtbalken 16 überstreichen, wie dies in Fig. 2 durch die Strahlen 10, 11, 12 innerhalb des Auges 4 gezeigt ist. Eine sogenannten Endo-Beleuchtung, ein dünner Lichtleiter, der in den hinteren Augenabschnitt eingeführt wird, der von Hand gehalten werden muß, ist daher nicht erforderlich.

Es sollte noch bemerkt werden, daß in den Fig. 1 und 2 nur ein optischer Beobachtungsstrahlengang 21 gezeigt ist. Bei den hier üblicherweise verwendeten Stereomikroskopen sind selbstverständlich zwei solcher Strahlengänge 21 vorhanden, die aber bei der Darstellung der Fig. 1 und 2 hintereinander liegen würden, so daß der zweite Strahlengang nicht gezeigt ist.

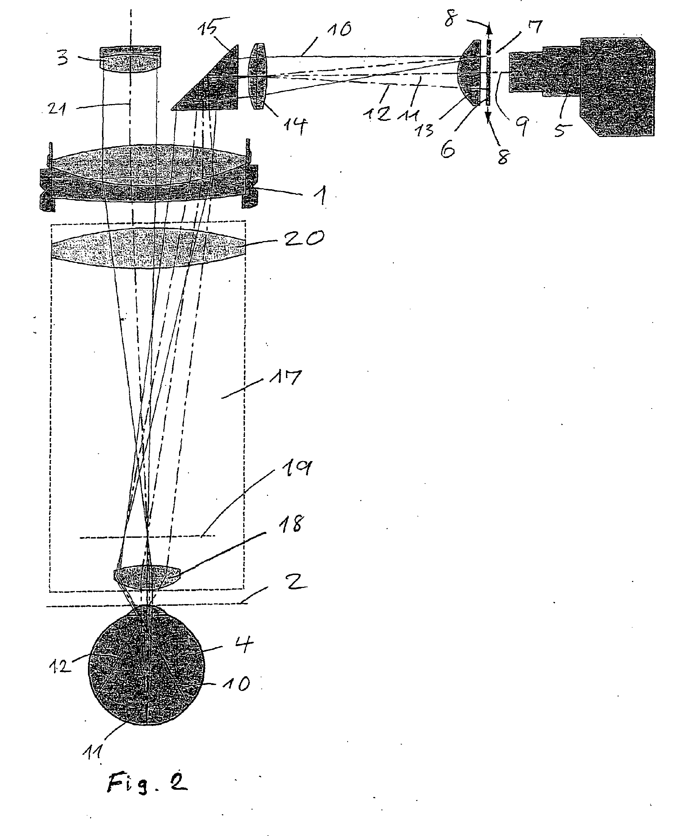

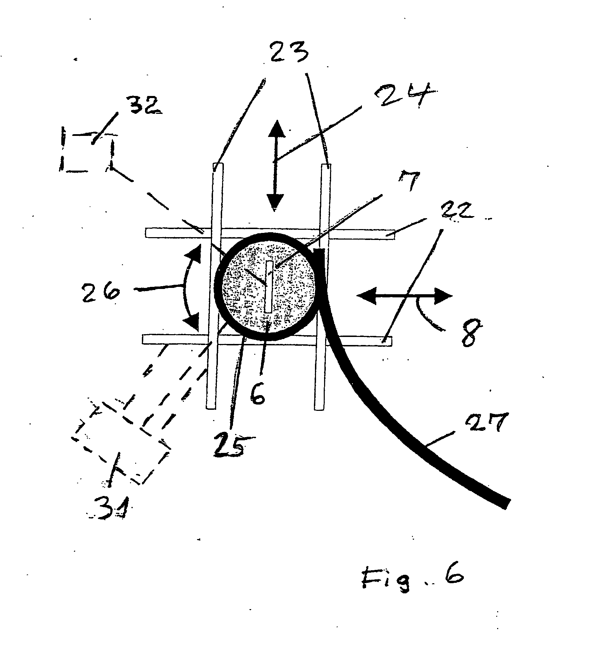

Wie dies in Fig. 6 gezeigt ist, ist bei einer Ausführungsform der Blendenträger 6 mit der Spaltblende 7 auf zwei senkrecht zueinander angeordneten Schlitten verschiebbar angeordnet, die bei 22 und 23 angedeutet sind. Dadurch ist nicht nur eine Bewegung in Richtung des Doppelpfeiles 8, sondern auch eine Bewegung in Richtung des Doppelpfeiles 24 möglich. Zusätzlich zur Anordnung auf diesem zweidimensionalen Lineartisch 22, 23 ist eine Drehung der Spaltblende 7 auf einem bei 25 angedeuteten Drehtisch in Richtung des Doppelpfeiles 26 möglich. Dieser Drehtisch 25 wird dabei über eine flexible Welle 27 eingestellt. Die entsprechenden Bewegungsmöglichkeiten sind auch in Fig. 3 gezeigt.

Fig. 4 zeigt eine andere Ausführungsform des Blendenträgers 6. Dieser ist kreisförmig und um eine Achse 28 in Richtung des Doppelpfeiles 29 drehbar. Die Achse 28 ist dabei zur optischen Achse 9 des Beleuchtungsstrahlenganges versetzt angeordnet, so daß sich bei Drehung in Richtung des Doppelpfeiles 29 ebenfalls im wesentlichen eine translatorische Bewegung ergibt, die allerdings von einer gewissen Schwenkbewegung des Spaltes 7 und damit des Lichtbalken 16 begleitet ist. Diese Schwenkbewegung ist aber bei genügend großem Abstand der Achsen 9 und 22 verhältnismäßig gering.

Bei der Ausführungsform der Fig. 4 ist gezeigt, daß auf dem Blendenträger 6 Spalte 7 unterschiedlicher Breiten angeordnet sind, so daß auch die Breite des Lichtbalkens 16 ausgewählt werden kann. Bei 30 ist noch ein teildurchlässiger Bereich des Blendenträgers 6 gezeigt. Dadurch wird das Auge 4 bzw. die Objektebene 2 nicht nur mit einem scharfen Lichtbalken 16 beleuchtet, sondern ein größerer Teil des Auges 4 bzw. der Objektebene 2 mit schwächerem diffusem Licht.

In Fig. 6 ist schematisch noch eine motorische Verstellung 31 in Richtung des Doppelpfeile 8, 24 und 26 sowie eine motorische Verstellung 32 der Spaltbreite angedeutet. Diese Verstellungen können z. B. durch Betätigung eines Fußschalters vorgenommen werden, der auf Elektromotoren in den Elementen 31, 32 wirkt.

## Patentansprüche

1. Operationsmikroskop mit einer die Objektebene (2, 19) mit einem Lichtfleck (16) beleuchtenden, hinter der Frontlinse (1) angeordneten Beleuchtungseinrichtung (13, 14, 15), in deren Strahlengang (9) eine denselben teilweise abdeckende Blende (6, 7) angeordnet ist, **dadurch gekennzeichnet, daß** die Blende (6, 7), eine Lichtquelle (5) der Beleuchtungseinrichtung und/oder ein Umlenkelemenent (15) für eine Verschiebung des Lichtflecks (16) mit einer translatorischen Bewegungskomponente in der Objektebene (2, 19) bewegbar sind.

2. Operationsmikroskop nach Anspruch 1, **dadurch gekennzeichnet, daß** die Blende (6, 7) in einem senkrecht zur optischen Achse (9) des Beleuchtungsstrahlenganges (4) verschiebbaren Blendenträger (6) angeordnet ist.

3. Operationsmikroskop nach Anspruch 1 oder 2, **dadurch gekennzeichnet, daß** die Blende (6, 7) in zwei zueinander senkrechten Richtungen (8, 24) linear senkrecht zur optischen Achse (9) des Beleuchtungsstrahlenganges verschiebbar ist.

4. Operationsmikroskop nach Anspruch 2 oder 3, **dadurch gekennzeichnet, daß** die Blende (6, 7) zusätzlich um eine zur optischen Achse (9) des Beleuchtungsstrahlenganges parallele Achse (28) drehbar ist.

5. Operationsmikroskop nach Anspruch 1, **dadurch gekennzeichnet, daß** die Blende (6, 7) in einem Blendenträger (6) angeordnet ist, der exzentrisch (bei 28) zur optischen Achse (9) des Beleuchtungsstrahlenganges drehbar gelagert ist.

6. Operationsmikroskop nach einem der Ansprüche 2 bis 5, **dadurch gekennzeichnet, daß** mehr als eine Blende (6, 7) auf dem Blendenträger (6) vorgesehen ist.

7. Operationsmikroskop nach einem der Ansprüche 1 bis 6, **dadurch gekennzeichnet, daß** die oder mindestens eine Blende (6, 7) spaltförmig ist.

8. Operationsmikroskop nach einem der Ansprüche 1 bis 7, **dadurch gekennzeichnet, daß** die oder mindestens eine Blende (6, 7) kreisförmig ist.

9. Operationsmikroskop nach einem der Ansprüche 1 bis 8, **dadurch gekennzeichnet, daß** die Größe der Blende (6, 7) (die Spaltbreite bzw. der Kreisdurchmesser) veränderbar ist.

10. Operationsmikroskop nach einem der Ansprüche 1 bis 9, **dadurch gekennzeichnet, daß** die Blende(n) auf einem wenigstens in Teilbereichen (30) teildurchlässigen Blendenträger angeordnet ist.

11. Operationsmikroskop nach einem der Ansprüche 1 bis 7, **dadurch gekennzeichnet, daß** die Blende(n) (6, 7) und/oder das Umlenkelement (15) motorisch verstellbar ist/sind.

## Claims

1. Operation microscope with an illuminating device (13, 14, 15) which is arranged behind the front lens (1) and illuminates the object plane (2, 19) with a light patch (16) and in whose beam path (9) a diaphragm (6, 7) is arranged which partially covers said beam path, **characterized in that** the diaphragm (6, 7), a light source (5) of the illuminating device and/or a deflection element (15) for moving the light patch (16) can be moved with a translatory movement component in the object plane (2, 19).

2. Operation microscope according to Claim 1, **characterized in that** the diaphragm (6, 7) is arranged in a diaphragm support (6) which can be moved perpendicular to the optical axis (9) of the illuminating beam path (4).

3. Operation microscope according to Claim 1 or 2, **characterized in that** the diaphragm (6, 7) can be moved in two directions (8, 24) perpendicular to one another and linearly perpendicular to the optical axis (9) of the illuminating beam path.

4. Operation microscope according to Claim 2 or 3, **characterized in that** the diaphragm (6, 7) can additionally be rotated about an axis (28) parallel to the optical axis (9) of the illuminating beam path.

5. Operation microscope according to Claim 1, **characterized in that** the diaphragm (6, 7) is arranged in a diaphragm support (6) which is rotatably mounted eccentrically (at 28) with respect to the optical axis (9) of the illuminating beam path.

6. Operation microscope according to one of Claims 2 to 5, **characterized in that** more than one diaphragm (6, 7) is provided on the diaphragm support (6).

7. Operation microscope according to one of Claims 1 to 6, **characterized in that** the diaphragm or at least one diaphragm (6, 7) is slit-shaped.

8. Operation microscope according to one of Claims 1 to 7, **characterized in that** the diaphragm or at least one diaphragm (6, 7) is circular.

9. Operation microscope according to one of Claims 1 to 8, **characterized in that** the size of the diaphragm (6, 7) (the slit width or circle diameter) can be modified.

10. Operation microscope according to one of Claims 1 to 9, **characterized in that** the diaphragm(s) is/are arranged on a diaphragm support which is partially transmitting at least in subareas (30).

11. Operation microscope according to one of Claims 1 to 7, **characterized in that** the diaphragm(s) (6, 7) and/or the deflection element (15) can be adjusted by motor.

## Revendications

1. Microscope chirurgical doté d'un dispositif d'éclairage (13, 14, 15) disposé derrière la lentille frontale (1), éclairant le plan de l'objet (2, 19) d'un point lumineux (16), dans le faisceau (9) duquel est disposé un diaphragme (6, 7) recouvrant en partie ledit faisceau, **caractérisé en ce que** le diaphragme (6, 7), une source de lumière (5) du dispositif d'éclairage et/ou un élément de déviation (15) pour un déplacement du point lumineux (16) sont mobiles dans le plan de l'objet (2, 19) avec une composante de déplacement en translation.

2. Microscope chirurgical selon la revendication 1, **caractérisé en ce que** le diaphragme (6, 7) est disposé dans un support de diaphragme (6) mobile perpendiculairement à l'axe optique (9) du faisceau d'éclairage (4).

3. Microscope chirurgical selon la revendication 1 ou 2, **caractérisé en ce que** le diaphragme (6, 7) est mobile dans deux directions (8, 24) perpendiculaires l'une à l'autre, de façon linéaire et perpendiculaire par rapport à l'axe optique (9) du faisceau d'éclairage.

4. Microscope chirurgical selon la revendication 2 ou 3, **caractérisé en ce que** le diaphragme (6, 7) peut être en plus amené en rotation autour d'un axe (28) parallèle à l'axe optique (9) du faisceau d'éclairage.

5. Microscope chirurgical selon la revendication 1, **caractérisé en ce que** le diaphragme (6, 7) est disposé dans un support de diaphragme (6), qui est logé de manière rotative et excentrée (en 28) par rapport à l'axe optique (9) du dispositif d'éclairage.

6. Microscope chirurgical selon l'une quelconque des revendications 2 à 5, **caractérisé en ce que** plus d'un diaphragme (6, 7) est prévu sur le support de diaphragme (6).

7. Microscope chirurgical selon l'une quelconque des revendications 1 à 6, **caractérisé en ce que** le ou au moins un diaphragme (6, 7) est en forme de fente.

8. Microscope chirurgical selon l'une quelconque des revendications 1 à 7, **caractérisé en ce que** le ou au moins un diaphragme (6, 7) est circulaire.

9. Microscope chirurgical selon l'une quelconque des revendications 1 à 8, **caractérisé en ce que** la dimension du diaphragme (6, 7) (la largeur de fente et/ ou le diamètre du cercle) peut être modifiée.

10. Microscope chirurgical selon l'une quelconque des revendications 1 à 9, **caractérisé en ce que** le(s) diaphragme(s) est(sont) disposé(s) sur un support de diaphragme au moins semi-transparent dans des zones partielles (30).

11. Microscope chirurgical selon l'une quelconque des revendications 1 à 7, **caractérisé en ce que** le(s) diaphragme(s) (6, 7) et/ou l'élément de déviation (15) est/sont réglable(s) de façon motrice.
